# EUROPEAN PATENT SPECIFICATION

(11) **EP 2 315 823 B1**
(45) Date of publication and mention of the grant of the patent: **07.03.2018**
(21) Application number: 09776231.4
(22) Date of filing: 03.07.2009
(51) Int. Cl.: C12M 1/18

(54) **ONE TO ONE IDENTIFICATION SYSTEM**
EINS-ZU-EINS-IDENTIFIZIERUNGSSYSTEM
SYSTÈME D'IDENTIFICATION INDIVIDUEL

(30) Priority: 05.07.2008 DK 200800949
(43) Date of publication of application: 04.05.2011
(73) Proprietor: Unisense FertiliTech A/S, 8200 Aarhus N (DK)
(72) Inventor: RAMSING, Niels, B., DK-8240 Risskov (DK); BERNTSEN, Jørgen, DK-8800 Viborg (DK); GUNDERSEN, Jens, K., DK-8260 Viby J (DK); PLOUGSGAARD, Holger Søe, DK-8250 Egå (DK)
(74) Representative: D Young & Co LLP
(86) International application number: PCT/DK2009/050162
(87) International publication number: WO 2010/003423

(56) References cited:
- WO-A1-98/05753
- DE-A1- 10 017 192
- JP-A- 2005 168 341
- JP-A- 2006 034 256
- US-A- 3 618 734
- US-A- 5 196 168
- US-A1- 2003 138 942
- US-A1- 2004 147 012
- US-A1- 2005 205 673
- US-B1- 6 228 636
- Anonymous: "Flow cytometry - Wikipedia", , 1 April 2017 (2017-04-01), XP055368455, Retrieved from the Internet: URL:https://en.wikipedia.org/wiki/Flow_cyt ometry [retrieved on 2017-05-02]

## Description

### Field of invention

The present invention relates to a system arranged for assisting secure handling of cells in order to minimize the risk of handling mistakes resulting in mix-up of cell samples.

### Background of invention

Culturing of in vitro fertilised embryos and other fragile cellular materials require sophisticated equipments in order to provide quality, traceability of cellular material and reproducibility of the culturing process. Temperature and atmosphere controls are basic elements and internal monitoring equipment is desired in order to minimize environmental changes during culturing and monitoring. Due to obvious reasons mix-up of individual cell samples, such as in vitro fertilised embryos should be prevented. In practice this problem is frequently solved by lowering the number of objects cultured in each incubator and in particular by culturing of only objects derived from one experimental setting, such as eggs from one woman, in one incubator, hereby ensuring one to one identification of objects and the origin of the objects. For practical and economical reasons this is unattractive as this limits the number of samples to be handled and/or requires a large numbers of culturing systems which is expensive and space consuming. US 2004/0147012 discloses a system for automatically processing cells in barcoded microtiter plates comprising an incubator.

### Summary of invention

The invention is defined by the appendant claims.

The present disclosure relates to a culturing system which allows for one to one identification of cells during the whole culturing period. This is obtained by providing a culturing system with loading/exit control means which ensures that only one device is capable of being loaded/removed at a time.

An aspect of the disclosure relates to a system arranged for performing culturing and optionally monitoring of at least two microscopic objects wherein said at least two microscopic objects are arranged in at least two separate devices, wherein said system comprises an incubator, a loading port for loading a device to be arranged in said incubator, and an exit port for exiting a device from said incubator, and wherein the system further comprises loading control means capable of controlling that only one device is capable of being loaded into the system at a time.

A further aspect of the disclosure relates to a device for monitoring and/or culturing of at least two microscopic objects, said device comprising a machine readable tag.

In a further aspect the disclosure relates to a method for handling of at least two microscopic objects comprising: culturing said at least two microscopic objects in at least two separate devices in an incubator, said incubator comprising loading control means capable of controlling that only one device is capable of being loaded in the system at a time.
An aspect of the invention relates to a system arranged for identifying a selected object in a device housing at least two microscopic objects wherein said system comprises means for providing information about a selected object to said device.
In a further aspect the invention relates to a method for handling a device comprising at least two microscopic objects, comprising selecting an object while the device is inside an incubator, wherein identification of said selected object is provided to said device.

### Definitions

Blastocyte: The blastocyte is a structure formed in early embryogenesis possessing an inner cell mass, or embryoblast which subsequently forms the embryo, and an outer cell mass, or trophoblast which forms the placenta. Blastocyte formation begins at day 5 and the blastocyte comprises 70-100 cells.

Embryo: A multi cellular diploid eukaryote in its earliest stage of development. In some cases the term "embryo" is used to describe a fertilized oocyte after implantation in the uterus until 8 weeks after fertilization at which stage it becomes a foetus. According to this definition the fertilized oocyte is often called a pre-embryo until implantation occurs. However, throughout this patent application we will use a broader definition of the term embryo, which includes the pre-embryo phase. It thus encompasses all developmental stages from the fertilization of the oocyte through morula, blastocyst stages hatching and implantation.

Zygote: The result of fertilization of an egg cell and a sperm cell is a cell called the zygote that includes all DNAfrom both parents.

### Detailed description of the invention

In the process of in vitro fertilization egg and sperm are incubated together in a culture media for about 18 hours, alternatively a single sperm is injected directly into the egg using intracytoplasmic sperm injection (ICSI). The fertilized egg shows two pronuclei and is subsequently passed to a special growth medium and left for about 48 hours until the egg has reached the 6-8 cell stage. At this time the selection of embryos is usually performed based on the quality and viability of embryo. The quality of embryos may be determined based on the visual appearance of the embryos, by taking features such as the number of cells, evenness of growth and degree of fragmentation into account. A method for selection of embryos, based on quality assessments of embryos, is described in PCT/DK2007/000291.
The timing of embryo transfer, which implicitly affects the duration of in vitro culturing of embryos are different in different regions, e.g. USA have programs with extended culturing, such as until the blastocyst stage, whereas embryos are normally transferred to the recipient uterus at the 6-8 cell stages in Europe (2-day transfer). The prolonged culturing may affect the selection criteria's for the embryos.
Culturing of embryos requires a system for performing culturing and optionally monitoring the embryos. Logically, the identity of embryos cultured in a given system should not be mixed up, as this could result in transfer of embryos to a wrong recipient. To prevent this, embryos from different individuals are usually cultured separately.

The present disclosure relates to a system arranged for culturing and optionally monitoring of microscopic objects, such as embryos. The system as described herein is arranged for culturing at least two microscopic object arranged in separate devices.
It is envisioned that the system may be applied for monitoring different objects, such as cell samples during culturing procedures. Applications can be such as live analysis during screening procedures where the selection parameter can be assessed using the monitoring equipment. The separate devices may thus be used to test different compounds, different concentrations or different cell types. If used in connection with a fluorescence microscope location of fluorescent tags can be viewed during incubation.
The system includes an incubator, which is adapted for cell culturing e.g. including any necessary regulatory features, such as temperature and atmosphere controls (see below). For quality assessment of embryos, as described above, it is advantageous to be able to monitor the embryos during culturing e.g. without having to remove the devices housing the embryos from the incubator avoiding any unnecessary disturbance or interference with cell development which is highly sensitive to environmental stress. Therefore it is preferred that the system includes monitoring equipment for optionally monitoring of the embryos during culturing.
By including monitoring equipment the necessary handling of the embryos may be minimized to loading and removing of the devices housing the microscopic objects. The microscopic objects to be cultured in the system is arranged in the devices by standard methods and subsequently loaded into the system through a loading port and removed through an exit port. To increase the safety of the system, e.g. to minimize the risk of any mix up of microscopic object, the system is equipped with loading control means capable of controlling that only one device is capable of being loaded into the system at a time.
Standard incubators include an opening which gives access to all samples in the incubator. Even if the user is following strict handling protocols and amble control routines it is physically possible to load or remove two or more samples or two or more racks containing such samples at one time increasing the risk of manual handling errors. By including loading control means the system of the present disclosure provides further security to the handling procedure of the embryos.

An aspect of the present disclosure relates to a system arranged for performing culturing and optionally monitoring of at least two microscopic objects wherein said at least two microscopic objects are arranged in at least two separate devices, wherein said system comprises an incubator, a loading port for loading a device to be arranged in said incubator, and an exit port for exiting a device from said incubator, and further comprises loading control means capable of controlling that only one device is capable of being loaded into the system at a time.

### Loading control means

The loading control means ensures that only one device is capable of being loaded into the system at a time. During use, embryos/fertilized eggs from one woman is to be located in one individual device and the following single loading procedure guarantees that only embryos derived from one potential mother is inserted into the incubator at a time. As described below each device may be provided with an identification tag to further facilitate identification of the device. In a preferred embodiment the size of the loading port allows entrance of one device at a time only. It is further preferred that the exit port allows exit of one device at a time only. In a most preferred embodiment the loading port and the exit port is the same port and only one device can be loaded/exit at a time restricted by the size of the loading/exit port. The identity of the device may be controlled by an identity tag, and the loading/exit may further be controlled by a computer which maintains control of the location of the individual devices.

In an alternative embodiment the storage means described herein below may include locks which fasten each device to a fixed position. These locks may be interconnected or computer controlled allowing only one lock to be opened at a time thereby providing loading control means. This may further be implemented by allowing only loading or removing of devices from one position, which is preselected. Loading of further devices will then require relocation of the first device. In this embodiment the loading and exit may be perform as the entry and exit of a ferris wheel. As above the location of individual devices and the loading of said individual devices in an unlocked position may be controlled by a computer to further minimize the risk that mix up of devices occur during handling of the devices.

### Storage means

To have the full benefit of the system according to the disclosure is it preferred that the system comprise storage means having two or more device holder positions, each device holder position being adapted to hold the device in a fixed position on the storage means. The storage means may be a conveyor belt for horizontal movements of devices. The storage means may thereby transfer each device to a monitoring position whereby it is possible to view the microscopic objects through the monitoring equipment.

In a preferred embodiment the storage means is capable of positioning a device relatively to a microscope to allow viewing of the microscopic objects (see below), by transferring the device to a monitoring position. It is further preferred that the device may be positioned to allow viewing of the individual object and/or allowing an overall view of the objects housed by a device (see section below relating to monitoring system).

Likewise it is further preferred that the storage means is capable of positioning a device or an empty device holder position in the loading port and/or exit port of the incubator. Thus in a preferred embodiment the system has a loading position and/or an exit position.

When the system is used in connection with an identification system as described herein below, it is further preferred that the storage means is capable of positioning a device relatively to a labelling equipment, by transferring of a device to a labelling position. The labelling position may be identical or different to one or more of the monitoring position, the loading or exit position. In a preferred embodiment the labelling position is identical or close to the exit position whereby an identification label is easily added just before removing of a device from the system. In a further preferred embodiment the labelling position is identical or close to the monitoring position, whereby an identification label is easily added at the end of monitoring of a device.

In a simple embodiment the storage means is a conveyor belt capable of moving the devices horizontally to allow both monitoring and loading/removing of devices. The devices may be placed on a slide which is moved sideways relative to the loading port. When the loading port is opened access to one device is provided.

A plurality of storage means comprising these features can be envisioned in accordance with the disclosure. A system including a transfer unit between a stacker (holding the devices) and an observation position is described in EP 1916296, and the storage means according to the disclosure may include one or more similar transfer unit(s), which can transfer a given device from a device holder to a monitoring position or loading/exit position. The devices are stored horizontally either side by side or at different heights inside the incubator. Different types of storage means may be combined to have a rearrangement system inside the incubator, such as conveyor belt, elevator type or ferris wheel type storage means. By including an internal rearrangement system the individual devices may be selected for monitoring or exit, and new devices may be loaded, while the other devices are maintained inside the incubator. It is contemplated that rearrangement of the devices inside the incubator may be performed manually from the outside of the incubator or preferably by an automated process controlled by a computer internally or externally of the system. The one to one identification of device, objects and the origin of objects, is assisted by the loading control means as described above. Identification tags (described below) may help to further ensure safety of the system, as the identity of the devices exiting or loaded into the system may be computer and/or manually controlled when individual device are handled.

In preferred embodiment the storage means comprises at least four, such as at least six device holder positions. In further embodiments the storage means comprise at least 8, such as at least 10 device holder positions.

### Identification tag

During use the devices for housing of the microscopic objects are provided with identification tags. Such a tag is preferably a machine readable identification tag, more preferably a barcode or an RFID (Radio-frequency identification). Use of machine readable tags is advantageously combined with a system provided with a machine reader capable of reading said machine readable identification tag.

### Device for housing microscopic objects

For culturing of embryos, a plurality of eggs are fertilized or cultured for fertilization, even if only a few are to be further processed. To optimise the usages of the system according to the disclosure, it is preferred that each device is capable of housing at least two microscopic objects such as at least five microscopic objects. In further embodiments it is even more preferred that each device is capable of housing at least 8 microscopic objects such as at least 10 microscopic objects.
In addition to the microscopic objects the device must further be able to contain the object media for culturing of said object. In a preferred embodiment each device comprises at least two depressions in a top surface of said device; each depression being adapted for housing a microscopic object and object media for culturing said microscopic object.

An aspect of the disclosure relates to a device for monitoring and/or culturing at least two microscopic objects which comprise a machine readable tag for identification of the device.
In an embodiment the device preferably comprises at least two depressions in a top surface of said device adapted for housing and individual microscopic object and object media.
In further preferred embodiment the device comprises an identification tag in the form of a barcode or a RFID.

As described above the security of the system is based on the expectation that eggs from different women are not cultured together in a device, even if the device has the capability to house a number of embryos as described above. This feature may alternatively be used to culture a number of embryos from the same woman in one device in the system. It is of cause also possible to arrange a number of devices housing embryos from the same woman in the system, to increase the chances of obtaining a reasonably number of high quality embryos suited for implantation.
In an embodiment each depression comprises an indent of a smaller cross section than the depression for housing each respective microscopic object and object media. Furthermore the devices according to the disclosure preferably exhibit a bottom surface profile corresponding to the contours of the device holders enabling easy and tight loading of the devices as this may be part of an orientation control system (see below). The device is preferably equipped with means for uniquely identifying each depression, such as depression markings. The markings are more preferably visible through the monitoring system (see below) allowing easy identification of a monitored depression.

Preferably the marking is directly visible through the microscope and sized to allow identification in the preferred view for assessment of the objects, meaning that the marking is probably microscopic in size to be viewed through the microscope, without interfering with the view of the object to be examined. Therefore the marking is most preferably located in the periphery of the depression. The marking may be a simple numbering system or a coordinate system or any other suitable system known in the art.

In a preferred embodiment said means for uniquely identifying each depression comprises at least one marker for identifying a row of depressions and at least one marker for identifying a column of depressions at said device.

In a preferred embodiment the depression marking is machine readable, which may be provided by a coordinate system as described above. A coordinate system is highly preferred as it allows different sized devices to be used.

### Information label and label receiving area

The device may further be construed for receiving an identification label by comprising a label receiving area. The label receiving area may be a common label receiving area or a depression specific label receiving area placed in connection with each depression. The label receiving area may be an area suitable for fastening of stickers or suitable for receiving simple written messages such as symbols, letters or colour signs, which may be used to communicate the information from a quality assessment.

A common label can be in the form of a label matching the upper or bottom surface of a device, whereby information regarding any selected embryos can be viewed during the further processing of the objects. The label is to inform the user about which objects have been selected. In an embodiment the information is in the form of an opening above the depressions housing the selected objects whereby the further processing is restricted to the selected objects. In a further embodiment the label is a cover overlaying the device housing the objects, where through access to only the selected objects is provided. In an even further embodiment colouring is used to mark depressions housing selected objects.

It is further possible to pre-attach labels which are heat or light sensitive to the device. These may be activated or made inactive by the culturing and monitoring system, during or at the end of the quality assessment.

In a preferred embodiment the device according to the disclosure comprises a label receiving area or a pre-attached label.
The use of an identification system as described herein below provides further safety to the handling procedure of microscopic objects. In further embodiment the device may comprise any of the features described below in relation to the identifying system.
Evaluation of the objects for further processing may, as described previously, be performed during culturing of the objects either manually by live monitoring or by analysing of photos or films acquired during the preceding culturing period or by computer assisted methods for determining object and particular embryo quality.
Upon selection of one or more objects for further processing it is preferred that the system provides means for labelling a depression housing a selected object, such as a depression specific label receiving area on the device housing the microscopic objects. Alternatively each device may comprise a common label receiving area where information on which depressions contain a selected object is deposited by the system.
The identification label may be any type of suitable label, such as a dot, a cross, a sticker, a cover with wholes or other more sophisticated means, such as a plate specially construed to receive a device housing the objects, where the plate is capable of pointing out the depressions containing the selected objects by use of light or other labelling methods. A common label may be such as schematic illustration of the device pointing out the selected depressions. The labelling may further be assisted by the identification tag and a computer based method for selecting the objects, whereby the system is minimally influenced by the individual handling the samples.

### Orientation control means

To further increase the safety of the system it is preferred that the system comprises orientation control means, such that loading of devices is only possible in one direction. Preferably device holders and devices are constructed to allow only loading of the devices in one orientation, by providing a device with a bottom surface which matches the receiving portion of the device holder. This may be even further improved by constructing pairs of device holders and devices which fit together uniquely, thereby also controlling which device is inserted into which device holder.

### Temperature control means

It is a prerequisite for cell culturing that temperature is continuously controlled in order to maintain a desired temperature inside the incubator and in particularly inside the devices housing the microscopic objects. In an embodiment the system according to the disclosure comprises a heat source suitable for providing temperatures for culturing said at least two microscopic objects.

As described above the devices housing the objects may in a preferred embodiment of the present system be held by the storage means, these may in a further preferred embodiment be used for ensuring thermal contact between said devices and said heat source. In an optimized more preferred embodiment said storage means comprises a device holder, which is adapted for matching a bottom surface provide of said device.

The system may accordingly be provided with a heat conducting layer adapted for providing a given stable temperature to at least said depressions, which temperature is suitable for cultivation, in particular suitable for incubation of said at least two microscopic objects.

### Monitoring system

The microscopic objects cultured in a system according to the disclosure are optionally monitored during culturing. Such monitoring may include visualisation of the objects by use of a microscope comprised by the system. This may be feasible by combining a system comprising a microscope with a system comprising storage means being arranged for positioning a device relative to the microscope thereby allowing at least one microscopic object to be viewed through the microscope. The device to be monitored is transferred to a microscope device holder position or a monitoring position. Depending on the desired view, the magnification of the system may be changed, to allow monitoring of each object individually or an overview of a whole device at one or more magnification(s).

Depending on the election criteria to be employed for evaluation of the microscopic objects, different automated procedures for monitoring and evaluation may be implemented. The microscope is preferably connected to a camera capable acquiring images of the microscopic object positioned to be viewed by the microscope. Images of individual microscopic objects may be acquired during the period of culturing the microscopic objects at regular intervals and used to elect objects for continued processing, such as in the case of embryos for implantation in a recipient uterus. Monitoring of objects may alternatively be performed using video equipment capable of acquiring live recordings.

The system of the disclosure preferably comprises a microscope and further imaging means, such as camera and/or video equipment.
As mentioned above any methods for electing the most promising embryos may be employed.

### Position control

Any monitoring of the microscopic objects by use of the microscope and/or any connected imaging means requires that the microscopic objects are arranged precisely relative to the microscope. To allow the microscopic objects to be viewed through/by the microscope and to ensure correct position of the device during culturing and monitoring it is preferred that the storage means and in particularly the microscope storage means comprises:
- at least one vertical hole extending through the device holder,
- a light emitter, such as a laser providing a vertical light, and
- a light sensor on the opposite side of the device relative to the light emitter for sensing the light being emitted when the device holder is in a correct position for monitoring and/or culturing.
It is further preferred that the system comprises storage means further comprising a control unit for automatic positioning of said device relative to said microscope and/or said heat source.

In a preferred embodiment the system according to the disclosure comprises a control unit adapted for controlling the temperature of said heat source for a stable incubator environment, and/or adapted for monitoring and/or acquiring images of said microscopic objects using said imaging means, and/or analyzing said images for a quality assessment of said objects, and/or keeping a record of replacement slides.

Different applications of the system according to the disclosure may benefit from non-invasive measurements of metabolic activity in the individual depressions holding the microscopic objects. The pH of media may change during culturing, and the gas content may change locally. Thus, in a more preferred embodiment the system comprises means for performing non-invasive measurements of metabolic activity comprising micro sensors for measuring e.g. pH, oxygen content and/or the like.

### Method for culturing microscopic objects

In a further aspect the present disclosure relates to a method for handling two microscopic objects comprising culturing said at least two microscopic objects in at least two separate devices in an incubator comprising loading control means capable of controlling that only one device is capable of being loaded in the system at a time.

It is preferred that the method comprises the step of culturing and optionally monitoring of said at least two microscopic objects by use of a system as described herein. It is further preferred that at least two devices as described herein is used for housing said at least two microscopic objects.

During regular quality assessment of embryos it is common that the device holding the embryos is removed from the incubator for quality assessment just prior to the selection, whereby the selection is performed on a single view of the objects at the end of the culturing period.

The system, devices and method described herein is particularly useful for culturing embryos prior to implantation, as the system is suited for performing quality assessments without interfering with the culturing environment. In a further preferred embodiment the microscopic objects are embryos, such as in vitro fertilized eggs.

It is further preferred that any or at least part of any quality assessment is performed while the embryos are inside the incubator.

By using a system according to the disclosure it is possible to base the quality assessment on several views of each individual embryo, such as at least two views. The objects may be examined at any time during the culturing period, and the analysis may be postponed by acquiring photos or even live movies of each embryo. The quality assessment may be performed with the aid of a computer, or by a computer alone based on some predetermined criteria / criterion.

The quality assessment may be used to select embryos for implantation, freezing and/or destruction based on different selection criteria.

It is preferred that the depressions comprising the selected embryos are identified and that this information is communicated to the user. In a preferred embodiment information about the selected embryos is provided by a label attached to the device, for example in the form of either a depression specific label or a common label including information on all embryos housed by the specific device.

In an alternative embodiment the information about the selected embryos is provided by activation or inactivation of a pre-attached label comprised by the device, such as a heat or light sensitive label.

In a further aspect the invention relates to a method for handling a device comprising at least two microscopic objects, said method comprising the step of selecting an object while the device is inside an incubator, wherein identification of said selected object is provided to said device.

### Identifying system

As described above an object may be selected manually or by a computerized method. As such microscopic objects may be very fragile it is desired to maintain the objects in the same environment as long as possible, e.g. that any selection is performed while the objects are maintained in a suitable incubator. Upon selection of an object, the object is to be further processed by various procedures depending on the type of objects housed by the system. Further processing most often involves removal of the object from the device housing said object. The selection process is preferably separate from the removal of the object and therefore the information regarding a selected object must be provided to the individual handling the further processing in a safe and easy way assisting removal of selected objects from the device.

The present application in a further aspect describes a system for identification of a selected object in a device housing at least two microscopic objects wherein said system comprises means for providing information about a selected object to said device.

The system according to the invention identifies, points out or marks the location of a selected object. In a preferred embodiment an object is selected based on a quality assessment. This is highly suited for e.g. selection of embryos for implantation.

As previously described the device housing the microscopic objects may be construed for receiving information regarding a selected object, by comprising a label receiving area. This area may be on a top surface of the device, a bottom surface of the device or alternatively on a device cover.

In one embodiment of the invention information about a selected object is provided by an information label on the information label receiving area, such as on the top surface of the device, on a bottom surface of the device or alternatively on a device cover.

To identify a selected object to the user of the system the information label providing the information about a selected object is preferably a visual mark. An information label may, as described previously, be in any suitable form, as long as the user is capable of using the information provided by the label. In a preferred embodiment the information about a selected object is provided by a visual mark, such a colour sign, a symbol or a written text. It is further preferred that such visual marks are visible during removal under microscope or when a step of selection control implies microscope inspection of selected objects.

In the specific embodiment wherein the information label is provided to a label receiving area of a device cover, the label may in a preferred embodiment be an opening in a device cover, which provides a physical labelling of selected objects or in other terms a physical hindrance obstructing the removal of unselected objects.

A label is preferably provided on an information label receiving area, either directly on said area or on a label attached to said area. As previously described pre-attached labels which may be heat or light sensitive may be used in accordance with the invention.

The identifying system may include further features to provide a system arranged for culturing and monitoring said at least two microscopic objects. A system thus preferably further comprises an incubator and monitoring equipment.

It is further envisioned that the identification system may comprise any of the features of the system arranged for performing culturing and optionally monitoring of at least two microscopic objects as described herein above.

In a further embodiment the identification system comprises a device for housing at least two microscopic objects as described herein. Thus, in a preferred embodiment the identification system is arranged for culturing and monitoring of more than one device.

By using features from one or more of the culturing system, the identifying system, the devices described herein, and the methods as described here above, a highly secure and functional system for treatment of microscopic objects is obtained. The security features includes the single loading preferably combined with an identification tag attached to the device linking each device with the origin of the microscopic objects housed by said device. Secondly, the selection of object may be performed while the objects are inside an incubator and the selection may further be based on several views of each object. In total this provides a basis for high quality selection. By using the identification system for linking the selected objects with its location in the device, by providing the information about a selected object to said device, the location of a selected object is clearly marked for further handling. In conclusion the system enables methods for securing one to one identification of the origin of objects the device holding said objects and an object selected there from.

### Detailed description of the drawings

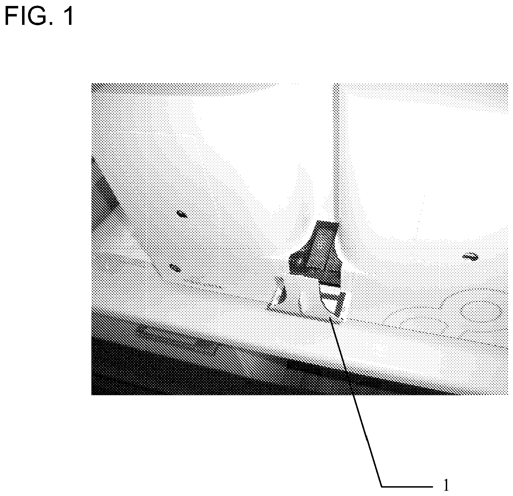

- **Figure 1:**: Loading control means. The incubator is equipped with a loading port (1) which provides access to only one object housing device. The picture reveals two devices inside the incubator, but only the leftmost device can be easily removed from the incubator. To remove further devices from the incubator, rearrangement of the devices by use of the storage means is required to transfer a second device to the device holder position directly inside of the loading port. Loading of devices must likewise occur one by one, and requires that an empty device holder position is present directly inside of the loading port.

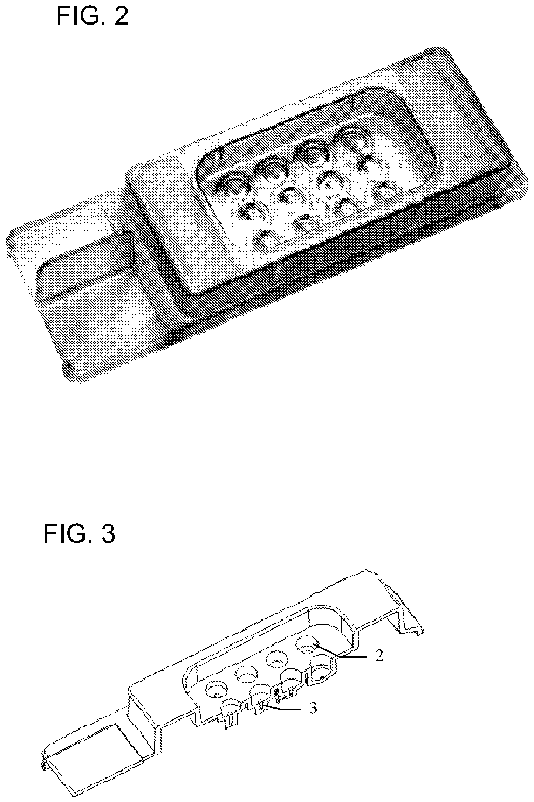

- **Figure 2:**: Device capable of housing 12 microscopic objects.
- **Figure 3:**: Side view of the device shown in figure 2, disclosing the depressions (2) and indents (3) of said depressions for accommodating the microscopic objects.

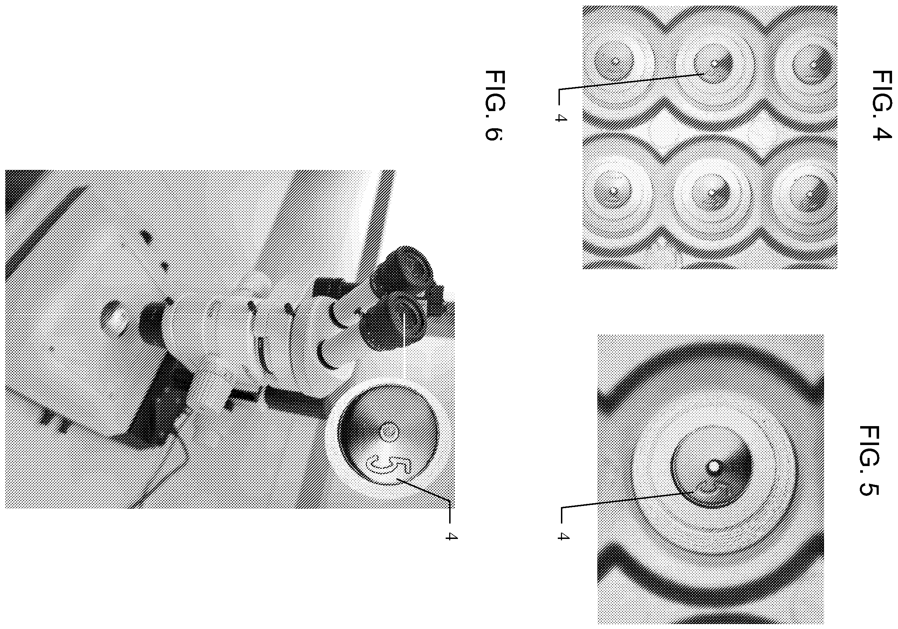

- **Figures 4-6:**: Identity markings (4) of individual depressions/indents shown at different magnifications.

## Claims

1. A system arranged for identifying a selected object in a device comprising depressions for housing at least two microscopic objects, each depression housing one of the at least two microscopic objects, wherein said system comprises means for providing information about a selected object to said device, said object preferably selected by a quality assessment.

2. The system according to claim 1, wherein the device comprises a label receiving area, such as a label receiving area located on a top surface, on a bottom surface of the device, said information about a selected object preferably provided by markings on the information label receiving area.

3. The system according to claims 1 to 2, wherein the means for providing information comprises a device cover, said device cover preferably comprising an information label receiving area, said information about a selected object are provide by a marking on the information label receiving area of the device cover, a marking such as an opening in the device cover.

4. The system according to any of claims 1 to 3, wherein information about a selected object is provided by a visual mark, such as a visual mark provided by colour, symbol or written text, the visual mark is preferably visible during removal of a selected object.

5. The system according to claim 4, where the mark is on an information label receiving area and/or on a label attached to an information label receiving area.

6. The system according to any of the claims 1 to 5, wherein the system is arranged for culturing and monitoring said at least two microscopic objects.

7. The system according to claim 6, wherein the system comprises an incubator and monitoring equipment.

8. The system according to any of the claims 1 to 7, wherein the system is arranged for culturing and monitoring of more than one device.

9. A method for handling a device comprising depressions for housing at least two microscopic objects, said method comprising the step of selecting an object while the device is inside an incubator, wherein identification of said selected object is provided to said device.

## Patentansprüche

1. System, das zur Identifizierung eines ausgewählten Objekts in einer Vorrichtung vorgesehen ist, die Vertiefungen zum Aufnehmen von mindestens zwei mikroskopischen Objekten umfasst, wobei jede Vertiefung eines der mindestens zwei mikroskopischen Objekte aufnimmt, wobei das System Mittel zum Bereitstellen von Informationen über ein ausgewähltes Objekt an die Vorrichtung umfasst, wobei das Objekt vorzugsweise durch eine Qualitätsbeurteilung ausgewählt wird.

2. System nach Anspruch 1, wobei die Vorrichtung einen Etikettempfangsbereich umfasst, wie einen Etikettempfangsbereich, der auf einer Oberseite, auf einer Unterseite der Vorrichtung angeordnet ist, wobei die Informationen über ein ausgewähltes Objekt vorzugsweise durch Markierungen auf dem Informationsetikettempfangsbereich bereitgestellt werden.

3. System nach den Ansprüchen 1 bis 2, wobei die Mittel zum Bereitstellen von Informationen eine Abdeckung der Vorrichtung umfassen, wobei die Abdeckung der Vorrichtung vorzugsweise einen Informationsetikettempfangsbereich umfasst, wobei die Informationen über ein ausgewähltes Objekt durch eine Markierung auf dem Informationsetikettempfangsbereich der Abdeckung der Vorrichtung, eine Markierung wie eine Öffnung in der Abdeckung der Vorrichtung, bereitgestellt werden.

4. System nach einem der Ansprüche 1 bis 3, wobei Informationen über ein ausgewähltes Objekt durch eine visuelle Markierung bereitgestellt werden, wie eine visuelle Markierung, die durch Farbe, Symbol oder geschriebenen Text bereitgestellt wird, wobei die visuelle Markierung vorzugsweise während der Entfernung eines ausgewählten Objekts sichtbar ist.

5. System nach Anspruch 4, wobei die Markierung sich auf einem Informationsetikettempfangsbereich und/oder auf einem Etikett befindet, das an einem Informationsetikettempfangsbereich angebracht ist.

6. System nach einem der Ansprüche 1 bis 5, wobei das System zum Kultivieren und Überwachen der mindestens zwei mikroskopischen Objekte vorgesehen ist.

7. System nach Anspruch 6, wobei das System einen Inkubator und Überwachungsgerätschaften umfasst.

8. System nach einem der Ansprüche 1 bis 7, wobei das System zum Kultivieren und Überwachen von mehr als einer Vorrichtung vorgesehen ist.

9. Verfahren zur Handhabung einer Vorrichtung, die Vertiefungen zum Aufnehmen von mindestens zwei mikroskopischen Objekten umfasst, wobei das Verfahren den Schritt des Auswählens eines Objekts umfasst, während sich die Vorrichtung im Inneren eines Inkubators befindet, wobei der Vorrichtung die Identifizierung des ausgewählten Objekts bereitgestellt wird.

## Revendications

1. Système agencé pour identifier un objet sélectionné dans un dispositif comprenant des creux pour loger au moins deux objets microscopiques, chaque creux logeant l'un des au moins deux objets microscopiques, dans lequel ledit système comprend un moyen de fourniture d'informations concernant un objet sélectionné audit dispositif, ledit objet étant de préférence sélectionné par une évaluation de qualité.

2. Système selon la revendication 1, dans lequel le dispositif comprend une zone de réception d'étiquette, telle qu'une zone de réception d'étiquette située sur une surface de dessus, sur une surface de dessous du dispositif, lesdites informations concernant un objet sélectionné étant de préférence fournies par des marquages sur la zone de réception d'étiquette d'informations.

3. Système selon les revendications 1 et 2, dans lequel le moyen de fourniture d'informations comprend un couvercle de dispositif, ledit couvercle de dispositif comprenant de préférence une zone de réception d'étiquette d'informations, lesdites informations concernant un objet sélectionné sont fournies par un marquage sur la zone de réception d'étiquette d'informations du couvercle de dispositif, un marquage tel qu'une ouverture dans le couvercle de dispositif.

4. Système selon l'une quelconque des revendications 1 à 3, dans lequel des informations concernant un objet sélectionné sont fournies par une marque visuelle, telle qu'une marque visuelle fournie par une couleur, un symbole ou un texte écrit, la marque visuelle étant de préférence visible pendant le retrait d'un objet sélectionné.

5. Système selon la revendication 4, dans lequel la marque est sur une zone de réception d'étiquette d'informations et/ou sur une étiquette fixée à une zone de réception d'étiquette d'informations.

6. Système selon l'une quelconque des revendications 1 à 5, dans lequel le système est agencé pour mettre en culture et surveiller lesdits au moins deux objets microscopiques.

7. Système selon la revendication 6, dans lequel le système comprend un incubateur et un équipement de surveillance.

8. Système selon l'une quelconque des revendications 1 à 7, dans lequel le système est agencé pour mettre en culture et surveiller plus d'un dispositif.

9. Procédé de manipulation d'un dispositif comprenant des creux pour loger au moins deux objets microscopiques, ledit procédé comprenant l'étape de sélection d'un objet tandis que le dispositif est à l'intérieur d'un incubateur, dans lequel une identification dudit objet sélectionné est fournie audit dispositif.
